# EUROPÄISCHE PATENTSCHRIFT

(11) **EP 1 648 342 B1**
(45) Veröffentlichungstag und Bekanntmachung des Hinweises auf die Patenterteilung: **20.04.2016**
(21) Anmeldenummer: 04763389.6
(22) Anmeldetag: 22.07.2004
(51) Int. Cl.: A61F 2/90

(54) **GEFLOCHTENER STENT ZUR IMPLANTATION IN EIN BLUTGEFÄSS**
WOVEN STENT TO BE IMPLANTED IN A BLOOD VESSEL
ENDOPROTHÈSE VASCULAIRE TRESSÉE DESTINÉE À ÊTRE IMPLANTÉE DANS UN VAISSEAU SANGUIN

(30) Priorität: 30.07.2003 DE 10335649
(43) Veröffentlichungstag der Anmeldung: 26.04.2006
(73) Patentinhaber: JOTEC GmbH, 72379 Hechingen (DE)
(72) Erfinder: KAUFMANN, Ralf, 72414 Rangendingen (DE); BOGENSCHÜTZ, Thomas, 72379 Hechingen-Stein (DE)
(74) Vertreter: Witte, Weller & Partner Patentanwälte mbB
(86) Internationale Anmeldenummer: PCT/EP2004/008172
(87) Internationale Veröffentlichungsnummer: WO 2005/011527

(56) Entgegenhaltungen:
- WO-A-99/25271
- WO-A-02/071975
- US-A- 5 741 333
- US-A- 5 836 966
- US-A1- 2003 130 611

## Beschreibung

Die vorliegende Erfindung betrifft einen Flechtstent zur Implantation in ein Blutgefäß, insbesondere in die Arteria carotis, mit einem in seiner Längsrichtung streckbaren, hohlen Körper, dessen Mantel ein Geflecht aus einer Vielzahl von fadenförmigen Elementen aufweist, die in expandiertem Zustand des Flechtstents eine Ebene senkrecht zur Längsrichtung unter einem Flechtwinkel schneiden.

Ein derartiger Flechtstent ist aus der DE 197 50 971 A1 bekannt.

Unter einem Stent versteht man eine radial expandierbare Endoprothese, die ein typisches intravaskuläres Implantat ist, das transluminal implantiert und radial vergrößert oder expandiert wird, nachdem es perkutan eingeführt wurde. Stents werden verwendet, um Blutgefäße zu verstärken und im vaskulären System eine Restenose nach einer vorhergehenden Angioplastie zu verhindern. Sie können selbstexpandierend sein oder durch eine von innen ausgeübte radiale Kraft expandiert werden, wenn sie beispielsweise auf einem Ballon montiert sind.

Der aus der DE 197 50 971 A1 bekannte Stent weist einen hohlzylindrischen Körper auf, dessen Außendurchmesser in etwa dem Innendurchmesser des Blutgefäßes entspricht, in das der Stent implantiert werden soll. Der Körper des Stents ist somit in Längsrichtung für den Durchtritt von Blut offen. Der Mantel ist aus mehreren gegeneinander versetzten fadenförmigen Elementen aufgebaut, die zu einem Geflecht mit einer Vielzahl polygonförmiger Zellen verflochten sind. Das Geflecht kann dabei so aufgebaut sein, dass zwei sich kreuzende Systeme der fadenförmigen Elemente so miteinander verkreuzt sind, dass jedes fadenförmige Element des einen Systems abwechselnd über und unter jedem fadenförmigen Element des anderen Systems geführt ist. Eine solche Bindung des Geflechts wird als Leinwand-Bindung bezeichnet.

Der bekannte Stent lässt sich auf einem Applikator in seiner Längsrichtung strecken, wodurch sich der Durchmesser des Stents zur Implantation verringern lässt. Mittels des Applikators wird der Stent im gestreckten bzw. gespannten Zustand in das Gefäß eingeführt. Nachdem der Stent an der gewünschten Stelle in dem Gefäß positioniert ist, wird der Applikator entfernt. Da nunmehr keine äußeren Längsdehnungskräfte auf den Stent wirken, relaxiert er auf Grund seiner zellenförmigen Struktur elastisch in seine ursprüngliche Länge, dehnt sich dabei radial aus und schmiegt sich an die Gefäßinnenwand an. Dies kann dadurch verstärkt werden, dass Materialien mit einem Formgedächtnis verwendet werden oder ein Ballonkatheter zur Unterstützung oder Bewirkung der Expansion verwendet wird.

Während es bekannt ist, derartige Flechtstents fortlaufend in der Art einer Meterware herzustellen, wird der aus der DE 197 50 971 A1 bekannte Flechtstent jeweils einzeln hergestellt, wozu die fadenförmigen Elemente während des Flechtvorganges an jedem Ende des Stents umgelenkt werden. Dadurch ist es möglich, dem Stent an seinem einen Ende eine sich trompetenartig erweiternde Krone zu vermitteln, durch die eine zusätzliche, besonders wirksame Verankerung des bekannten Stents in dem Blutgefäß erreicht wird.

Von Nachteil bei derartigen Flechtstents ist allgemein, dass sie eine große Längenänderung bei der Streckung erfahren, wobei die Längenänderung um so größer ist, je größer der ursprüngliche Durchmesser und je kleiner der ursprüngliche Flechtwinkel sind. Die entsprechend umgekehrte Längenverkürzung beim Expandieren des Flechtstents wird jedoch häufig als nachteilig angesehen. Die Positionierung eines Stents an der gewünschten Stelle in dem Blutgefäß ist nämlich ein kritischer Faktor, der die Wirkung des Stents und den Erfolg des medizinischen Eingriffs maßgeblich bestimmt. Da der Bereich in dem Blutgefäß, in dem der Stent expandiert werden soll, üblicherweise für einen Mediziner schwer zugänglich ist, ist es wichtig, dass der Durchmesser und die Länge des Stents im expandierten Zustand genau bekannt sind, damit er präzise positioniert werden kann.

Ein weiteres mit Flechtstents verbundenes Problem besteht darin, dass die Radialkraft und Steifigkeit schon bei geringer Elongation des Stents stark nachlässt, so dass die genaue Dimensionierung und Positionierung eines Flechtstents kritischer ist als bei Stents, die sich bei der Expansion nicht verkürzen. Ein derartiger Stent ist beispielsweise in der US 6,106,548 beschrieben.

In der US 5,741,333 ist ein selbstexpandierender Stent zur Einführung in geeignete Hohlräume des menschlichen Körpers offenbart. Der hierin offenbarte Stent weist einen schlauchförmigen Körper mit geflochtenen Filamenten auf, die einen mittleren Abschnitt mit einem ersten Durchmesser bilden und die zwei glockenförmig aufgeweitete Endabschnitte mit einem Durchmesser bilden, der zum Ende ansteigt.

Die WO 99/25271 offenbart einen Stent zur Implantation im menschlichen Körper, welcher einen hohlzylindrischen Körper aufweist, der aus mehreren umfänglich gegeneinander versetzten fadenförmigen Elementen aufgebaut ist. Die fadenförmigen Elemente sind zu einem Geflecht verflochten, und weisen in allen Bereichen des Stents den gleichen Flechtwinkel auf. WO 99/2571 offenbart alle Merkmale des Oberbegrifs des Anspruchs 1.

Die US 5,836,966 offenbart einen Stent mit variierender nach außen gerichteter Radialkraft. Diese unterschiedlichen Radialkräfte werden dadurch erreicht, dass in Zonen, wo eine verstärkte Radialkraft gewünscht ist, eine erhöhte Materialdichte vorgesehen ist.

Flechtstents werden daher heutzutage nicht eingesetzt, wenn es darauf ankommt, in einem sehr genau definierten Bereich eines Blutgefäßes eine große Radialkraft auszuüben, um einer Restenose entgegenzuwirken.

Ein relativ neues Einsatzgebiet für Stents ist die perkutane Behandlung von Läsionen der A. carotis nach perkutaner Angioplastie. Die auf diese Weise behandelten Stenosen in der A. carotis externa entstehen durch Arteriosklerose der Gefäßwand. Dabei entsteht eine harte, brüchtige Innenschicht, die den Blutstrom mehr und mehr einengt und dadurch die Versorgung des Gehirns mindert. Bei einem Verschluss kommt es zu einem weitläufigen Schlaganfall der betroffenen Hirnhälfte mit nachfolgenden irreversiblen Hirnschäden oder sogar Exitus.

Die Hauptkomplikation bei perkutaner Angioplastie mit nachfolgender Stentimplantation wird jedoch durch die Ablösung von Partikeln der brüchigen Innenschicht, sog. Plaques, ausgelöst, die als Emboli in Hirnregionen eingeschwemmt werden, wo sie einen lokalen Schlaganfall mit z.T. schweren irreversiblen Hirnschäden auslösen können.

Sofern der Zustand des Gefäßes es ermöglicht, wird daher heutzutage auf eine "Vordilation" des Gefäßes durch einen Ballonkatheter verzichtet, sondern es wird unmittelbar ein selbstexpandierender Stent perkutan und transluminal in den Bereich der Stenose appliziert. Besonders kritisch dabei ist jedoch die Ausdehnungsphase der Stents. Nach dem Einführen und Freisetzen in der Stenose entwickeln diese Stents oft nicht genügend Radialkraft, um ihre vorgefertigte Form zu erreichen. Deshalb wird nach dem Platzieren des Stents ein transluminaler Angioplastie-Ballon in den halb entfalteten Stent eingeführt und aufgeblasen, um Stent und Stenose aufzuweiten. Dabei kann es zu den bereits erwähnten iatrogenen Plaqueablösungen kommen, die nach Deflation des Ballons als Emboli in das Gehirn geschwemmt werden.

Um das Auftreten von Schlaganfällen zu verhindern, werden deshalb sog. zerebrale Protektionssysteme eingesetzt, mit denen die ersten Emboli abgefangen und entfernt werden. Ein solches System ist das PercuSurge System von PercuSurge Inc., Sunnyvale, CA, USA. Diese zerebralen Protektionssysteme bringen jedoch eine weitere Intervention mit häufig klinischen und symptomatischen Implikationen für die Patienten mit sich. Ferner können sich aber bereits gelockerte Plaqueteilchen auch noch mehrere Tage nach der Stentimplantation ablösen und durch die Maschen eines herkömmlichen Stents in den Blutstrom gelangen und so schwere Schlaganfälle verursachen.

Um dieses Problem zu lösen, schlägt die EP 1 101 456 A1 einen Stent vor, der in seinem mittleren Abschnitt mit einem biokompatiblen, elastischen Material ummantelt ist und nach der Stentimplantation zwischen der Endoprothese und der Wand des Blutgefäßes zu liegen kommt. Auf diese Weise wird das thrombogene Material an der Wand gehalten, wodurch verhindert wird, dass sich Plaques ablösen und in den Blutstrom gelangen.

Dieser "ummantelte" Stent weist jedoch insbesondere für den vorgeschlagenen Anwendungsfall eine ganze Reihe von Nachteilen auf. Zum einen verhindert die ihn umgebende elastische Membran eine Verankerung des Stents in der Wand des Blutgefäßes, so dass die Gefahr besteht, dass er seine Position verändert und seine Schutzwirkung verliert. Ferner ist der bekannte Stent teuer und kompliziert in der Herstellung, was auf die zusätzlich erforderliche elastische Membran zurückzuführen ist.

Auch die eingangs erwähnte US 6,106,548 beschäftigt sich mit dem Problem der Plaqueablösung und deren Transport als Emboli in das Gehirn.

Der bekannte Stent besteht aus einer Vielzahl von Ringen, die jeweils aus V-Streben bestehen. Benachbarte Ringe sind durch wellenartige Verbindungsteile miteinander verbunden, die jeweils an Scheitelpunkten von V-Streben in benachbarten Ringen befestigt sind. Diese Verbindungsteile gleichen die Längenänderungen der Ringe beim Expandieren aus, so dass der Stent seine Länge nicht verändert, wenn er in dem Blutgefäß expandiert wird. Diese Druckschrift erwähnt ferner, dass der Außendurchmesser des bekannten Stents geringfügig größer sein soll als der Innendurchmesser des Blutgefäßes, um den Stent sicher an der gewünschten Position zu verankern und zu verhindern, dass er sich aus dieser Position wegbewegt.

Die durch die V-Streben und Verbindungsteile gebildeten Maschen können in verschiedenen Abschnitten unterschiedliche Maschenweiten aufweisen, wobei kleinere Maschen verwendet werden, um die Ablösung von Plaques zu verhindern.

Der bekannte Stent kann so ausgelegt sein, dass er im expandierten Zustand Bereiche mit unterschiedlichen Außendurchmessern aufweist, so dass er sich an Gefäße oder Abzweigungen anpassen kann, wo die Lumendurchmesser variieren, wie es beispielsweise an der Carotisbifurcation der Fall ist.

Der bekannte Stent wird aus einem Röhrchen mit Laserschneidtechnik gefertigt oder aus vorfabrizierten V-Streben und Verbindungsteilen hergestellt, die anschließend miteinander beispielsweise durch Schweißen verbunden werden.

Wegen der Forderung, dass sich dieser Stent bei der Expansion nicht verkürzen soll, ist er nur auf technisch anspruchsvolle Weise herzustellen, so dass seine Produktion sehr kostenintensiv ist.

Ein sich ebenfalls nur geringfügig bei der Expansion verkürzender Stent ist aus der US 5,938,697 bekannt. Diese Druckschrift beschäftigt sich mit dem Problem, dass Stents mit über ihrer Länge gleicher Radialkraft zwar ein Blutgefäß im Stenosebereich offen halten können, jedoch an den äußeren Abschnitten stärker als erforderlich auf gesunde Gefäßbereiche drücken. Vergleichbare Probleme werden in konischen Gefäßbereichen, beispielsweise an der Carotisbifurcation, gesehen.

Der bekannte Stent löst dieses Problem dadurch, dass er längs seiner Längserstreckung variierende radiale Kräfte ausübt und eine unterschiedliche Steifigkeit besitzt.

Zu diesem Zweck ist der Stent aus ringförmigen Abschnitten aus serpentinenartigen Segmenten aufgebaut, die zickzackförmig um den Abschnitt verlaufen. Die einzelnen Ringe sind untereinander dadurch verbunden, dass alle oder einige benachbarte Zickzacksegmente an ihren Scheitelpunkten miteinander durch Streben verbunden sind. Durch die Abmaße dieser Streben sowie die Anzahl der Streben zwischen zwei Ringen wird eine offenere oder geschlossenere Struktur erreicht, weil weniger oder mehr Metallanteil zwischen den Ringen vorhanden ist. Die Ringe selbst sind identisch. Auf diese Weise soll erreicht werden, dass das Verhältnis von Metallfläche zu Blutgefäßfläche über der Länge des Stents konstant ist.

Die sog. geschlossene Struktur weist der Stent in den Bereichen auf, in denen eine gute Steifigkeit und Plaqueabdeckung erreicht werden soll, während die sog. offene Struktur dort zu finden ist, wo mehr Flexibilität erreicht werden soll.

In einem Ausführungsbeispiel weist der Stent einen mittleren Abschnitt auf, in dem er eine sehr hohe radiale Kraft ausübt, während die radiale Kraft in den sich anschließenden distalen und proximalen Abschnitten deutlich geringer ist.

Bei diesem Stent ist von Nachteil, dass er keinen effizienten Schutz gegen Plaqueablösung bildet, auch der übermäßig hohe Druck auf die Gefäßinnenwand im Stenosebereich kann nicht verhindern, dass sich kleinste Plaqueteilchen ablösen und durch die Maschen in den einzelnen Ringen in den Blutstrom gelangen.

Vor diesem Hintergrund liegt der vorliegenden Erfindung die Aufgabe zugrunde, einen Stent zu schaffen, der sich einerseits sicher im Stenosebereich verankert und andererseits effektiv die Ablösung von Plaques bzw. deren Eintritt in die Blutbahn als Emboli verhindert.

Bei dem eingangs genannten Flechtstent wird diese Aufgabe erfindungsgemäß dadurch gelöst, dass er in einem mittleren Abschnitt einen kleineren Flechtwinkel aufweist als in seinen sich in Längsrichtung an den mittleren Abschnitt anschließenden distalen und proximalen Abschnitten, und dass der mittlere Abschnitt im expandierten Zustand einen kleineren Außendurchmesser aufweist als der distale und der proximale Abschnitt.

Die der Erfindung zugrunde liegende Aufgabe wird auf diese Weise vollkommen gelöst.

Die Erfinder der vorliegenden Anmeldung haben nämlich erkannt, dass die der Erfindung zugrunde liegende Aufgabe gerade mit einem Flechtstent gelöst werden kann, wenn dessen Nachteile gegenüber anderen Stentstrukturen bewusst ausgenutzt werden. Durch den kleinen Flechtwinkel ist der erfindungsgemäße Flechtstent insbesondere in dem mittleren Abschnitt zwar stark streckbar, was eigentlich unerwünscht ist, er weist dort in seinem expandierten Zustand jedoch gerade deshalb eine große Dichte auf und ist dort auch besonders steif, kann also Kräfte von außen gut aufnehmen. Auf Grund seiner großen Dichte und Steifigkeit in seinem mittleren Abschnitt bietet der neue Flechtstent einen guten Schutz gegen Plaqueablösung und verhindert mit seinen engen Maschen in diesem Abschnitt ferner, dass sich dennoch ablösende Plaques in die Blutbahn gelangen.

Der neue Flechtstent kann darüber hinaus unabhängig von seiner Form und den lokalen Flechtwinkeln in einen langgestreckten Katheder geladen werden, der mindestens eine luminale Querschnittsfläche aufweist, die der Summe aller Flechtdrahtquerschnitte entspricht.

Bei dem erfindungsgemäßen Flechtstent ist ferner von Vorteil, dass der Durchmesser im mittleren Abschnitt so gewählt wird, dass sich die Flechtmaschen dort völlig aufstellen und ihren Winkel in Richtung des vorgegebenen Flechtwinkels verkleinern können, wobei sich der Flechtstent dabei insgesamt deutlich verkürzt. Wegen des etwas kleineren Durchmessers wird im mittleren Abschnitt auch die extreme Verkürzung bei der Expansion verringert. Durch den etwas kleineren Durchmesser wird außerdem der Druck auf die Stenose reduziert und der Flechtstent bleibt dicht. Andererseits reagiert der dichte mittlere Abschnitt fast wie ein dünnwandiges Rohrstück mit geschlossenem Mantel, wenn eine äußere Kompression auftritt. Dabei verspannen sich der distale und der proximale Abschnitt mit ihren Drahtenden in den Gefäßwänden. Wegen ihres größeren Außendurchmessers üben also der distale und der proximale Abschnitt einen deutlich größeren radialen Druck auf die Gefäßinnenwand aus als der mittlere Abschnitt. Die Erfindung geht also gerade den umgekehrten Weg, wie er in der eingangs diskutierten US 5,938,697 vorgeschlagen wird.

Ferner wendet sich die Erfindung von dem in der eingangs diskutierten US 6,106,548 vorgeschlagenen Konzept ab, wonach ein im Bereich der Carotisbifurcation einzusetzender Stent sich bei der Expansion nicht verkürzen darf.

Nach Erkenntnis der Erfinder verspannt sich der neue Flechtstent an seinen distalen und proximalen Abschnitten derart, dass eine mögliche Verlängerung und somit eine Rückstellung des mittleren kleinen Flechtwinkels verhindert wird. Auf diese Weise wird im Bereich der Stenose eine hohe Steifigkeit aufrecht erhalten und gleichzeitig ein sehr dichtes Geflecht geschaffen, das den Austritt von Emboli verhindert. Idealerweise entspricht der Innendurchmesser des Stents im Bereich der mittleren Zone dem maximalen Durchmesser des PTA-Ballons (Perkutaner Transluminaler Angioplastie-Ballon) und somit der beabsichtigten Aufweitung der Stenose. Durch diese Wahl der Durchmesser wird verhindert, dass im mittleren Abschnitt eine nicht vollständig expandierte Stentzone entsteht, die an Engmaschigkeit und Schutzwirkung einbüßen würde. Durch den größeren Flechtwinkel und Durchmesser im distalen und proximalen Abschnitt stellen sich dort die Drahtenden besser gegen die distal und proximal der Stenose liegenden Gefäßwände auf und der Stent formt sich dort weicher an.

Dabei ist es bevorzugt, wenn der Flechtstent in seinem expandierten Zustand einen Außendurchmesser aufweist, der sich von seinem proximalen zu seinem distalen Ende kontinuierlich verändert, vorzugsweise verringert, wobei weiter vorzugsweise der Außendurchmesser im mittleren Abschnitt verglichen mit einer kontinuierlichen Veränderung nochmals verringert ist.

Auf diese Weise wird ein konischer Stent geschaffen, der im mittleren Abschnitt eine größere Dichte aufweist als in den beiden äußeren Abschnitten. Auf diese Weise ist der Stent besonders gut zum Einsatz in der A. carotis interna geeignet.

Wenn der Außendurchmesser im mittleren Abschnitt kleiner ist als durch die Konusform vorgegeben, so ergeben sich auch bei einem konischen Stent die oben bereits diskutierten Vorteile, die mit dem verringerten Durchmesser im mittleren Abschnitt einhergehen.

Allgemein ist es bevorzugt, wenn der Flechtstent am distalen und proximalen Abschnitt einen Außendurchmesser aufweist, der größer ist als der Innendurchmesser des Blutgefäßes an der Stelle, wo der jeweilige Abschnitt nach der Implantation liegt.

Bei dieser Maßnahme ist von Vorteil, dass sich der Flechtstent distal und proximal von der Stenose sehr fest in der Gefäßinnenwand verspannt, so dass die Lage des mittleren Abschnittes gut fixiert ist. Damit ist es nicht zwingend erforderlich, dass der mittlere Abschnitt sich selbst in seiner Lage fixiert, dies wird durch die beiden äußeren Abschnitte bewirkt. Wie bereits erwähnt, führt diese Fixierung des mittleren Abschnittes dazu, dass der kleine Flechtwinkel und damit die Dichtigkeit im mittleren Abschnitt beibehalten wird.

Allgemein ist es bevorzugt, wenn der Flechtstent im mittleren Abschnitt einen Außendurchmesser aufweist, der gleich oder geringfügig kleiner ist als der aufdilatierte Innendurchmesser des Blutgefäßes an der Stelle, wo der mittlere Abschnitt nach der Implantation liegt.

Bei dieser Maßnahme ist von Vorteil, dass sich der mittlere Abschnitt vollständig expandieren kann, so dass die fadenförmigen Elemente den vorgegebenen Flechtwinkel wieder einnehmen können, sich also aufstellen und für eine große Dichtigkeit sowie entsprechende Steifigkeit im mittleren Abschnitt sorgen.

Allgemein ist es bevorzugt, wenn das Geflecht eine Leinwand-Bindung aufweist.

Bei dieser Art des Geflechtes sind die sich kreuzenden schraubenlinienförmigen Fadenlagen derart miteinander verkreuzt, dass jedes fadenförmige Element des einen Systems abwechselnd über und unter jedem fadenförmigen Element des anderen Systems geführt ist. Die sich ergebenden polygonförmigen Maschen sind bei diesem Flechtmuster rhombenförmig ausgebildet. Diese Art des Geflechtes hat sich für Stents zur Implantation in Blutgefäße als besonders geeignet erwiesen.

Ferner ist es bevorzugt, wenn die fadenförmigen Elemente aus einem Material mit Formgedächtniseffekt, insbesondere aus Nitinol, bestehen.

Bei dieser Maßnahme ist von Vorteil, dass der neue Flechtstent auf Grund seiner Superelastizität nach dem Freisetzen an der Stenosestelle seine ursprüngliche Form selbsttätig wieder annimmt, die Aufweitung insbesondere im Bereich der Stenose kann dabei aber durch einen Ballonkatheter weiter unterstützt werden, um sicherzustellen, dass sich die Maschen im mittleren Abschnitt vollständig aufstellen.

Ferner ist es bevorzugt, wenn der Flechtstent mit einer medizinisch wirksamen Substanz beschichtet ist.

Diese Maßnahme ist an sich bekannt, die medizinisch wirksamen Substanzen können beispielsweise eine Restenose verhindern, ein Abheilen von Verletzungen der Gefäßinnenwand beschleunigen oder die Ausbildung von Entzündungen verhindern.

Ferner ist es bevorzugt, wenn der Flechtstent an seinen beiden Enden eine sich konisch aufweitende Krone aufweist.

Bei dieser Maßnahme ist von Vorteil, dass der neue Flechtstent über seine beiden Enden noch effektiver in der Gefäßinnenwand verankert wird. Durch die Expansion des freigesetzten Flechtstents, die ggf. durch einen Ballonkatheter unterstützt wird, verkürzt sich der Flechtstent insbesondere wegen des kleinen Flechtwinkels im mittleren Abschnitt stark, so dass die äußeren Enden sich auf den mittleren Abschnitt zu bewegen. Durch den von außen auf den Flechtstent wirkenden Druck neigt er nach Deflation des Ballons dazu, sich wieder zu verlängern, was durch die aufgeweiteten Kronen verhindert wird. Die Kronen können als zusätzliche oder alternative Maßnahme zu einer Vergrößerung des Außendurchmessers am distalen und proximalen Abschnitt über den Innendurchmesser des Blutgefäßes hinaus verwendet werden.

Die Erfindung betrifft ferner die Verwendung des erfindungsgemäßen Stents bei einem Aneurysma.

Unter einem Aneurysma versteht man eine Ausweitung oder Aussackung eines arteriellen Blutgefäßes infolge angeborener oder erworbener Gefäßwandveränderungen. Die Aussackung kann dabei die Gefäßwand als Ganzes erfassen oder es kann bei einem sog. falschen Aneurysma Blut aus dem Lumen des Gefäßes zwischen die Gefäßwandschichten treten und diese auseinanderscheren. Die Nichtbehandlung eines Aneurysmas kann im fortgeschrittenen Stadium zu einer Ruptur des Blutgefäßes führen, woraufhin der Patient innerlich verbluten kann.

Aneurysmen treten häufig im Bereich der Baucharterie oder Brustarterie, ferner jedoch auch in den Bereichen des aufsteigenden oder absteigenden Astes der Aorta sowie in Hirnarterien auf.

Die Erfinder haben erkannt, dass der Stent auf extrem kleine Durchmesser geflochten werden kann, wodurch er für Anwendungen auch in sehr kleinen Gefäßen, beispielsweise in den Hirnarterien, eingesetzt werden kann.

Bei einem Einsatz in einem peripheren Gefäßaneurysma, beispielsweise in Hirnarterien, kann der mittlere Abschnitt derart platziert werden, dass sich dieser Abschnitt ohne radial wirkende Wanddrücke ungehindert verdichten kann. Da der mittlere Abschnitt einen kleineren Flechtwinkel aufweist als die sich in der Längsrichtung des Stents anschließenden distalen und proximalen Abschnitte, bildet sich in dem Bereich des mittleren Abschnitts eine dichte Zone, durch die kein oder lediglich wenig Blut in den Bereich des Aneurysmas gelangt. Über den proximalen und distalen Abschnitt des Stents wird dieser in den Bereichen des Gefäßes, die nicht vom Aneurysma betroffen sind, fest verankert, so dass sämtliches Blut in den Flechtstent geführt wird und diesen am Aneurysma vorbei am distalen Ende wieder verlässt. Danach dienen also die durchlässigeren Endbereiche des Stents zum einen der axialen Fixierung in gesunden Gefäßabschnitten und erlauben andererseits die Durchblutung abgehender Gefäße am Rande des Aneurymas. Durch den mittleren Abschnitt, der auf Grund seines kleinen Flechtwinkels eine dichte Zone bildet, wird das Blut im Aneurysma vom Austausch mit dem Kreislauf nahezu ausgeschlossen. Dadurch können sich im Aneurysma Thromben bilden, wodurch dessen weiteres Wachstum gestoppt wird. Mit dem mittleren dichten Abschnitt wird dabei gleichzeitig verhindert, dass die im Bereich des Aneurysmas gebildeten Thromben in den Blutstrom gelangen. Gleichzeitig wird jedoch die Durchblutung des Hauptgefäßes und nah abgehender Gefäße nicht beeinträchtigt.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachstehenden Beschreibung näher erläutert. Es zeigen:
- Fig. 1: die geometrische Situation an der linken Carotisbifurcation beim Menschen;
- Fig. 2: einen konischen Flechtstent zur Implantation in die A. carotis interna aus Fig. 1;
- Fig. 3: einen zylindrischen Flechtstent zur Implantation in die A. carotis communis aus Fig. 1;
- Fig. 4: ein Blutgefäß mit Stenose und eingesetztem Flechtstent aus Fig. 3;
- Fig. 5: das Blutgefäß aus Fig. 4, mit eingesetztem PTA-Ballon;
- Fig. 6: das Blutgefäß aus Fig. 5, mit expandiertem PTA-Ballon;
- Fig. 7: das Blutgefäß aus Fig. 6 mit entferntem PTA-Ballon und vollständig expandiertem Flechtstent; und
- Fig. 8: ein Blutgefäß mit Aneurysma und eingesetztem Flechtstent aus Fig. 3.

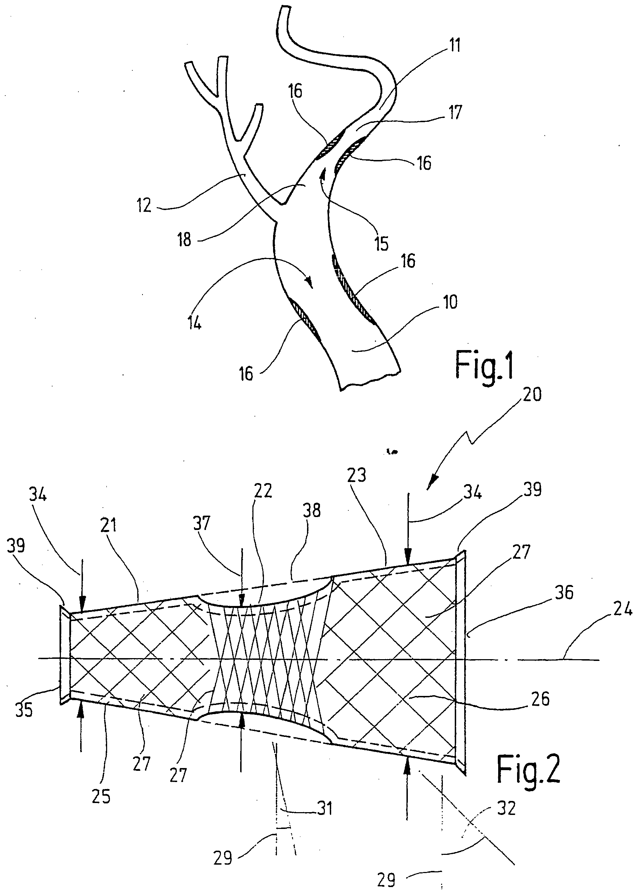

In Fig. 1 ist die geometrische Situation an der Carotisbifurcation bei einem Menschen gezeigt. Die A. carotis communis 10 geht hier in die A. carotis interna 11 über, wobei sich die A. carotis externa 12 abzweigt. In der A. carotis communis ist eine Stenose 14 und in der A. carotis interna 11 eine Stenose 15 durch harte, brüchige Bereiche 16 angedeutet. Es ist zu erkennen, dass die A. carotis interna 11 in einem Bereich 17 distal von der Stenose 15 einen geringeren Innendurchmesser aufweist als in einem Bereich 18 proximal von der Stenose 15.

In Fig. 2 ist ein erfindungsgemäßer Flechtstent 20 gezeigt, der in den Bereich der Stenose 15 in Fig. 1 eingesetzt werden soll. Der Flechtstent 20 weist einen distalen Abschnitt 21, einen mittleren Abschnitt 22 sowie einen proximalen Abschnitt 23 auf, die sich in Längsrichtung 24 des Flechtstents 20 aneinander reihen.

Der Flechtstent 20 hat einen hohlen Körper, dessen bei 25 angedeuteter Mantel aus einem Geflecht 26 von fadenförmigen Elementen 27 besteht, die im vorliegenden Fall Nitinol-Draht sind. Das Geflecht 26 ist in Leinwand-Bindung ausgeführt, wie es in der eingangs erwähnten DE 197 50 971 A1 beschrieben ist.

In Fig. 2 ist der Flechtstent 20 in seinem vollständig expandierten Zustand, also in dem Fertigungszustand dargestellt, in dem im mittleren Abschnitt 22 die fadenförmigen Elemente 27 eine Ebene 29 senkrecht zur Längsrichtung 24 unter einem Flechtwinkel 31 schneiden, der deutlich kleiner ist als der Flechtwinkel 32 im distalen und proximalen Abschnitt 21 bzw. 23.

Der Flechtstent 20 weist einen Außendurchmesser 34 auf, der sich von seinem proximalen Ende 36 zu seinem distalen Ende 35 kontinuierlich verringert, so dass er insgesamt eine konische Struktur aufweist, die der konischen Form der A. carotis interna 11 im Bereich der Stenose 15 aus Fig. 1 entspricht.

Im mittleren Abschnitt 22 ist der Außendurchmesser 37 verglichen mit der bei 38 gestrichelt angedeuteten Konusform verringert. Mit diesem mittleren Abschnitt 22 mit verringertem Außendurchmesser 37 kommt der Flechtstent 20 aus Fig. 2 im Bereich der brüchigen Bereiche 16 der Stenose 15 zu liegen, wobei der distale Abschnitt 21 im distalen Bereich 17 der A. carotis interna zu liegen kommt.

Durch den kleinen Flechtwinkel 31 im mittleren Abschnitt 22 weist der Flechtstent 20 dort eine sehr dichte Maschenstruktur auf, die die Bereiche 16 gegen die Innenwand der A. carotis interna 11 drückt und verhindert, dass sie sich ablösen bzw. in den Blutstrom gelangen.

Im Bereich des distalen Abschnittes 21 sowie proximalen Abschnittes 23 ist der Außendurchmesser 34 des Flechtstents 20 größer als der entsprechende Innendurchmesser der A. carotis interna 11, während der Außendurchmesser 37 etwa gleich dem Innendurchmesser der A. carotis interna 11 im Bereich der Stenose 15 nach entsprechender Dilation ist.

Auf diese Weise verspannt sich der Flechtstent 20 mit seinem distalen und seinem proximalen Abschnitt 21 bzw. 23 in der A. carotis interna und verhindert, dass sich der mittlere Abschnitt 22 verlängert, was zu einer Vergrößerung des Flechtwinkels 31 führen würde. Der Flechtstent 20 wird jedoch im mittleren Bereich 22 mit aufgestellten Flechtmaschen im Flechtwinkel 31 gehalten, so dass der mittlere Abschnitt 22 nicht nur eine sehr dichte Struktur aufweist, sondern darüber hinaus eine entsprechende Steifigkeit, die eine weitere Einschnürung im Bereich der Stenose 15 verhindert.

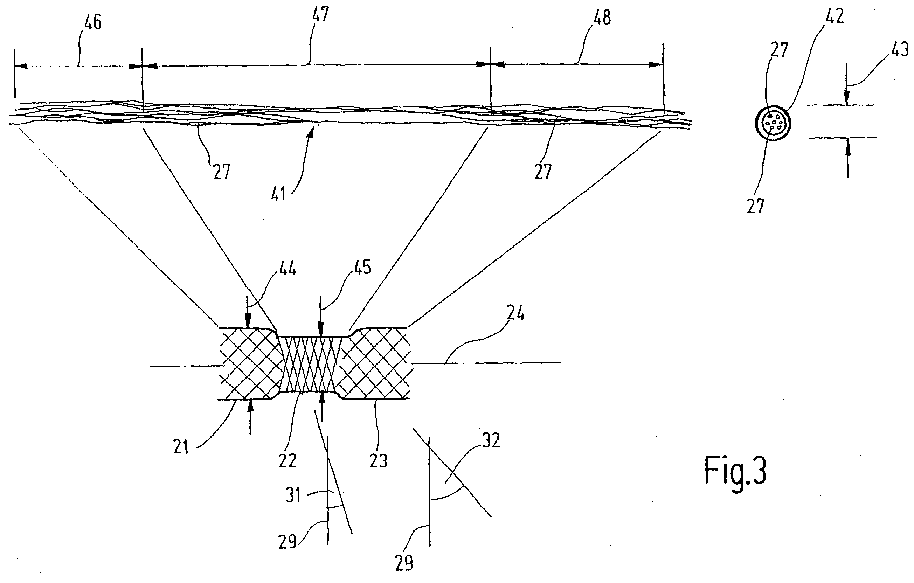

In Fig. 3 ist ein Flechtstent 41 gezeigt, der in den Bereich der Stenose 14 aus Fig. 1 eingesetzt werden soll. Der Flechtstent 41 ist unten in Fig. 3 im vollständig expandierten Zustand dargestellt, wobei zu erkennen ist, dass er eine hohlzylindrische Form aufweist. Oben in Fig. 3 ist der Flechtstent 41 in seiner langgestreckten Form gezeigt, in den er in einen in Fig. 3 rechts schematisch angedeuteten Katheter 42 geladen ist, dessen Innendurchmesser 43 so gewählt ist, dass er eine luminale Querschnittsfläche aufweist, die der Summe der Querschnittsflächen der Flechtdrähte 27 entspricht.

Der Flechtstent 41 weist in seinem distalen und proximalen Abschnitt 21 bzw. 23 einen Außendurchmesser 44 auf, der etwas größer ist als der Innendurchmesser der A. carotis communis im Bereich der Stenose 14. In seinem mittleren Abschnitt 22 weist der Flechtstent 41 einen verringerten Außendurchmesser 45 auf, der etwa dem Innendurchmesser des Flechtstents bei den Bereichen 16 nach entsprechender Dilation entspricht.

Wie schon bei dem Flechtstent 20 weist auch der Flechtstent 41 im mittleren Abschnitt 22 einen Flechtwinkel 31 auf, der kleiner ist als der Flechtwinkel 32 im distalen und proximalen Abschnitt 21 bzw. 23.

Auf Grund des kleinen Flechtwinkels 31 streckt sich der mittlere Abstand 22 deutlich weiter als der distale und proximale Abstand 21 und 23. Im vollständig gestreckten Zustand, wie er in Fig. 3 oben gezeigt ist, ist die Länge 46 des distalen Abschnittes 21 um ca. 30 bis 60 % größer als im expandierten Zustand. Gleiches gilt für die Länge 48 des proximalen Abschnittes 23.

Auf Grund des sehr viel kleineren Flechtwinkels 31 beträgt die Länge 47 des mittleren Abschnittes 22 im gestreckten Zustand etwa 500 - 600 % von der Länge im expandierten Zustand, wie er in Fig. 3 unten beschrieben ist.

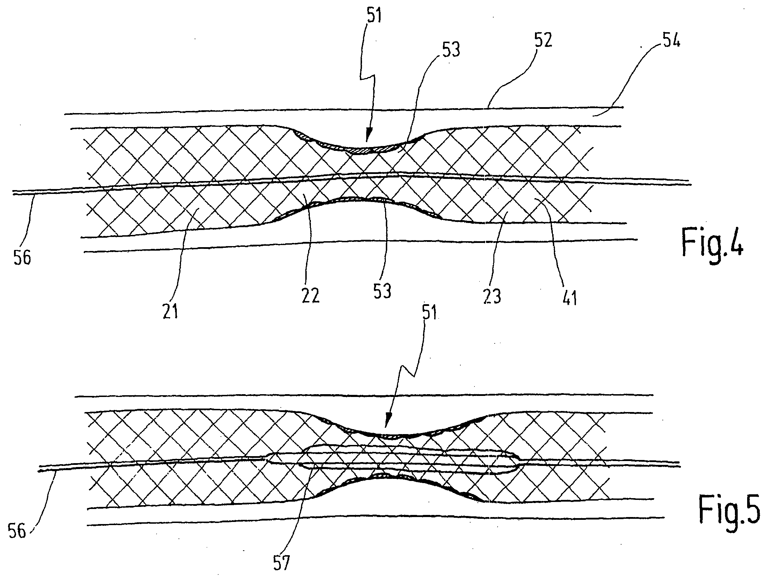

In Fig. 4 ist jetzt in schematischer Seitenansicht eine Stenose 51 in einem Blutgefäß 52 gezeigt, bei dem sich Plaques 53 an der Gefäßwand 54 angesammelt haben, wobei die Gefäßwand 54 im Bereich der Stenose 51 darüber hinaus eingeschnürt ist. In Fig. 4 ist der Flechtstent 41 aus Fig. 3 bereits in den Bereich der Stenose 51 eingeführt worden, so dass der mittlere Abschnitt 22 im Bereich der Einschnürung und Plaques 53 liegt, während der distale und proximale Abschnitt 21 und 23 distal und proximal von der Stenose 51 liegen. Der Flechtstent 41 wurde in üblicher Weise mit dem in Fig. 3 gezeigten Katheter 42 eingeführt und danach freigesetzt, so dass er sich in die in Fig. 4 gezeigte Form entspannen konnte. Insbesondere wenn die fadenförmigen Elemente 27 aus Nitinol-Draht bestehen, also ein Formgedächtnis aufweisen, expandiert sich der Flechtstent 41 in seine aufgeprägte Grundform. Allerdings reicht die Expansionskraft des Flechtstents 41 u.U. nicht aus, um insbesondere im mittleren Abschnitt 22 die Flechtmaschen so weit aufzustellen, dass der Flechtwinkel 31 eingenommen wird.

Zu diesem Zweck wird mit Hilfe eines Führungsdrahtes 56 ein PTA-Ballon 57 in den Bereich der Stenose 51 eingeführt, wie es in Fig. 5 gezeigt wird.

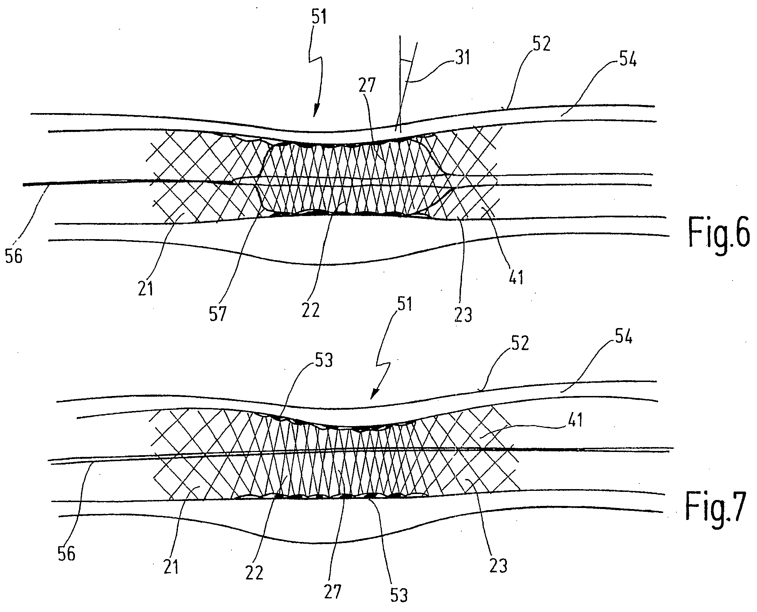

Der PTA-Ballon 57 wird dann aufgeblasen, wodurch sich die Verengung im Bereich der Stenose aufweitet, wie es in Fig. 6 gezeigt ist. Idealerweise wird der PTA-Ballon so weit aufgeblasen, bis er einen Außendurchmesser aufweist, der dem Innendurchmesser des Flechtstents 41 im Bereich des mittleren Abschnittes 22 in seiner Grundform entspricht, wenn also die fadenförmigen Elemente 27 wieder den Flechtwinkel 31 erreicht haben. Die Situation ist in Fig. 6 dargestellt. Der Außendurchmesser des Flechtstents 41 im mittleren Abschnitt 22 ist jetzt etwa gleich dem aufdilatierten Innendurchmesser des Blutgefäßes 52 im Bereich der Stenose 51. Verglichen mit Fig. 5 ist zu sehen, dass der Flechtstent 41 sich insbesondere in den Abschnitten 21 und 23 infolge einer Art "scherenförmiger Verkleinerung" deutlich verkürzt hat.

Nach Deflation wird der PTA-Ballon 57 entfernt, so dass sich die in Fig. 7 gezeigte Situation ergibt, in der im mittleren Abschnitt 22 des Flechtstents 41 die fadenförmigen Elemente 27 ein dichtes Maschennetz bilden, durch das die Plaques 53 innen an der Gefäßwand 54 festgehalten werden, so dass sie nicht in den Blutstrom gelangen können.

Bei der Expansion des Flechtstents 41 mit Hilfe des PTA-Ballons 57 verkürzt sich der Flechtstent 41, seine äußeren Bereiche bewegen sich sozusagen auf die Stenose 51 zu. Durch den durch die Gefäßwand 54 auf den Flechtstent 41 ausgeübten Druck wird dieser jetzt an dem distalen und proximalen Abschnitt 21 und 23 innen an der Gefäßwand 54 verankert, so dass im mittleren Abschnitt 22 der Flechtwinkel 31 erhalten bleibt, die erforderliche Dichtheit des Geflechtes sowie Steifigkeit also gewahrt bleibt.

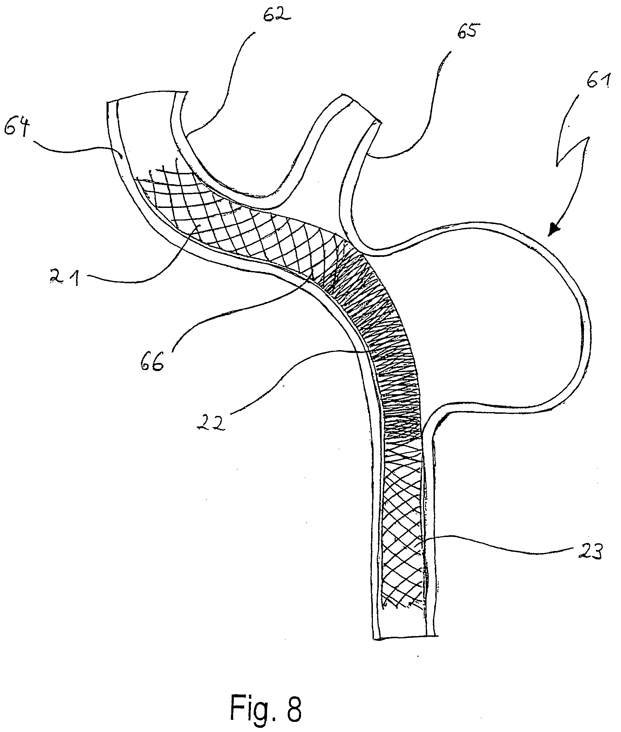

In Fig. 8 ist in schematischer Seitenansicht ein Aneurysma 61 in einem Blutgefäß 62 sowie einem Nebengefäß 65 gezeigt. Die Gefäßwand 64 des Gefäßes 62 ist im Bereich des Aneurysmas 61 auseinandergeschert. In Fig. 8 ist der Flechtstent 66 im Bereich des Aneurysmas 61 eingeführt worden, so dass der mittlere Abschnitt 22 im Bereich der auseinandergescherten Gefäßwände 62 des Aneurysmas 61 liegt, während der distale und proximale Abschnitt 21 und 23 distal und proximal vom Aneurysma 61 liegen. Der Flechtstent 66 wurde in üblicher Weise wie bereits für Fig. 4 beschrieben in das Gefäß eingeführt und freigesetzt, so dass er sich in die in Fig. 8 gezeigte Form entspannen konnte.

In Fig. 8 ist zu erkennen, dass der mittlere Bereich eine dichte Zone bildet, durch welche das im Gefäß geführte Blut nicht in den Bereich des Aneurysmas 61 gelangen kann, sondern vielmehr über den proximalen Abschnitt 23 im Gefäß weitergeleitet wird. Gleichzeitig gewährleistet der distale Abschnitt 21, dessen Flechtstruktur weniger dicht ist als die des mittleren Abschnittes 22, dass Blut aus dem Flechtstent in diesem Bereich austreten kann, wodurch das Nebengefäß 65 mit Blut versorgt bleibt und gleichzeitig der Stent 66 sicher im Gefäß 62 verankert bleibt. Durch den mittleren, dichten Abschnitt 22 wird das Blut im Aneurysma 61 vom Austausch mit dem Kreislaufblut nahezu ausgeschlossen, wodurch eine Thrombenbildung im Aneurysma 61 erreicht werden kann. Gleichzeitig verhindert der Flechtstent 66 durch seine Lage im Gefäß 62, dass im Aneurysma 61 gebildete Thromben in das Gefäß 62 gelangen und ggf. in anschließenden Gefäßen Embolien auslösen könnten.

## Patentansprüche

1. Flechtstent zur Implantation in ein Blutgefäß (52, 10, 11, 62), insbesondere in die Halsschlagader (Arteria carotis), mit einem in seiner Längsrichtung (24) streckbaren, hohlen Körper, dessen Mantel (25) ein Geflecht (26) aus einer Vielzahl von fadenförmigen Elementen (27) aufweist, die im expandierten Zustand des Flechtstents (20, 41, 66) eine Ebene (29) senkrecht zur Längsrichtung (24) unter einem Flechtwinkel (31, 32) schneiden,
**dadurch gekennzeichnet, dass** der Flechtstent (20, 41, 66) in einem mittleren Abschnitt (22) einen kleineren Flechtwinkel (31) aufweist als in seinen sich in Längsrichtung (24) an den mittleren Abschnitt (22) anschließenden distalen und proximalen Abschnitten (21, 23), und dass der mittlere Abschnitt (22) im expandierten Zustand einen kleineren Außendurchmesser (45) aufweist als der distale und der proximale Abschnitt (21, 23).

2. Flechtstent nach Anspruch 1, **dadurch gekennzeichnet, dass** er in seinem expandierten Zustand einen Außendurchmesser (34) aufweist, der sich von seinem proximalen zu seinem distalen Ende (36, 35) kontinuierlich verändert, vorzugsweise verringert, wobei der Außendurchmesser (37) im mittleren Abschnitt (22) verglichen mit einer kontinuierlichen Veränderung (38) noch mal verringert ist.

3. Flechtstent nach einem der Ansprüche 1 oder 2, **dadurch gekennzeichnet, dass** er am distalen und proximalen Abschnitt (21, 23) einen Außendurchmesser (34, 44) aufweist, der größer ist, als der Innendurchmesser des Blutgefäßes (51, 10, 11, 62) an der Stelle (14, 15), wo der jeweilige Abschnitt (21, 23) nach der Implantation liegt.

4. Flechtstent nach einem der Ansprüche 1 bis 3, **dadurch gekennzeichnet, dass** er im mittleren Abschnitt (22) einen Außendurchmesser (37, 45) aufweist, der gleich oder geringfügig kleiner ist als der aufdilatierte Innendurchmesser des Blutgefäßes (51, 10, 11, 62) an der Stelle (14, 15), wo der mittlere Abschnitt (22) nach der Implantation liegt.

5. Flechtstent nach einem der Ansprüche 1 bis 4, **dadurch gekennzeichnet, dass** das Geflecht (26) eine Leinwandbindung aufweist.

6. Flechtstent nach einem der Ansprüche 1 bis 5, **dadurch gekennzeichnet, dass** die fadenförmigen Elemente (27) aus einem Material mit Formgedächtniseffekt, insbesondere aus Nitinol bestehen.

7. Flechtstent nach einem der Ansprüche 1 bis 6, **dadurch gekennzeichnet, dass** er mit einer medizinisch wirksamen Substanz beschichtet ist.

8. Flechtstent nach einem der Ansprüche 1 bis 7, **dadurch gekennzeichnet, dass** er an seinen beiden Enden (35, 36) eine sich konisch aufweitende Krone (39) aufweist.

## Claims

1. A braided stent to be implanted in a blood vessel (52, 10, 11, 62), in particular in the carotid artery (Arteria carotis), with a hollow body which is stretchable in its longitudinal direction (24) and whose circumferential surface (25) has a braid (26) comprising a multiplicity of filamentary elements (27) which, in the expanded state of the braided stent (20, 41, 66), intersect a plane (29), perpendicular to the longitudinal direction (24), at a braiding angle (31, 32), **characterized in that** the braided stent (20, 41, 66) has a smaller braiding angle (31) in a central portion (22) than in its distal and proximal portions (21, 23) which adjoin the central portion (22) in the longitudinal direction (24), and that the central portion (22), in the expanded state, has a smaller external diameter (45) than the distal and the proximal portions (21, 23).

2. The braided stent as claimed in claim 1, **characterized in that**, in its expanded state, it has an external diameter (34) which changes continuously, preferably decreases, from its proximal end (36) to its distal end (35), wherein the external diameter (37) in the central portion (22) is reduced still further compared to a continuous change (38).

3. The braided stent as claimed in one of claims 1 or 2, **characterized in that**, at the distal and proximal portions (21, 23), it has an external diameter (34, 44) which is greater than the internal diameter of the blood vessel (51, 10, 11, 62) at the site (14, 15) where the respective portion (21, 23) lies after the implantation.

4. The braided stent as claimed in one of claims 1 through 3, **characterized in that**, in the central portion (22), it has an external diameter (37, 45) which is equal to or slightly smaller than the dilated internal diameter of the blood vessel (51, 10, 11, 62) at the site (14, 15) where the central portion (22) lies after the implantation.

5. The braided stent as claimed in one of claims 1 through 4, **characterized in that** the braid (26) has a plain weave.

6. The braided stent as claimed in one of claims 1 through 5, **characterized in that** the filamentary elements (27) are made from a material with a shape-memory effect, in particular from nitinol.

7. The braided stent as claimed in one of claims 1 through 6, **characterized in that** it is coated with a medically active substance.

8. The braided stent as claimed in one of claims 1 through 7, **characterized in that** it has a conically widening crown (39) at its two ends (35, 36).

## Revendications

1. Endoprothèse vasculaire tressée destinée à l'implantation dans un vaisseau sanguin (52, 10, 11, 62), en particulier dans la carotide (arteria carotis), comprenant un corps creux étirable dans sa direction longitudinale (24), dont l'enveloppe (25) présente un treillis (26) constitué d'une pluralité d'éléments en forme de fils (27), qui, dans l'état expansé de l'endoprothèse vasculaire tressée (20, 41, 66), coupent un plan (29) perpendiculairement à la direction longitudinale (24) suivant un angle de tressage (31, 32),
**caractérisée en ce que** l'endoprothèse vasculaire tressée (20, 41, 66) présente, dans une section centrale (22), un angle de tressage (31) plus petit que dans ses sections distale et proximale (21, 23) se raccordant à la section centrale (22) dans la direction longitudinale (24), et **en ce que** la section centrale (22), dans l'état expansé, présente un diamètre extérieur (45) plus petit que celui des sections distale et proximale (21, 23).

2. Endoprothèse vasculaire tressée selon la revendication 1, **caractérisée en ce qu'**elle présente, dans son état expansé, un diamètre extérieur (34) qui varie, de préférence diminue, en continu depuis son extrémité proximale jusqu'à son extrémité distale (36, 35), le diamètre extérieur (37) dans la section centrale (22) étant encore réduit par comparaison avec une variation continue (38).

3. Endoprothèse vasculaire tressée selon l'une quelconque des revendications 1 ou 2, **caractérisée en ce qu'**elle présente, au niveau des sections distale et proximale (21, 23), un diamètre extérieur (34, 44) qui est plus grand que le diamètre intérieur du vaisseau sanguin (51, 10, 11, 62) à l'emplacement (14, 15) où est située la section respective (21, 23) après l'implantation.

4. Endoprothèse vasculaire tressée selon l'une quelconque des revendications 1 à 3, **caractérisée en ce qu'**elle présente, dans la section centrale (22), un diamètre extérieur (37, 45) qui est identique ou légèrement inférieur au diamètre intérieur dilaté du vaisseau sanguin (51, 10, 11, 62) à l'emplacement (14, 15) où est située la section centrale (22) après l'implantation.

5. Endoprothèse vasculaire tressée selon l'une quelconque des revendications 1 à 4, **caractérisée en ce que** la tresse (26) présente une armure toile.

6. Endoprothèse vasculaire tressée selon l'une quelconque des revendications 1 à 5, **caractérisée en ce que** les éléments (27) en forme de fils sont constitués d'un matériau ayant un effet à mémoire de forme, en particulier de nitinol.

7. Endoprothèse vasculaire tressée selon l'une quelconque des revendications 1 à 6, **caractérisée en ce qu'**elle est revêtue d'une substance à effet thérapeutique.

8. Endoprothèse vasculaire tressée selon l'une quelconque des revendications 1 à 7, **caractérisée en ce qu'**elle présente au niveau de ses deux extrémités (35, 36) une couronne (39) s'élargissant coniquement.
